# EUROPEAN PATENT APPLICATION

(11) **EP 4 163 927 A1**
(43) Date of publication of application: **12.04.2023**
(21) Application number: 21200969.0
(22) Date of filing: 05.10.2021
(51) Int. Cl.: G16H 20/70, G16H 50/20, A61B 5/16, A61B 5/00, G16H 10/60

(54) **CONTINUOUS MONITORING TO DETECT CHANGES IN A USER'S MENTAL STATE TO IMPLEMENT STEPPED CARE**

(71) Applicant: Koa Health B.V., 08018 Barcelona (ES)
(72) Inventor: Garriga Calleja, Roger, 08470 Barcelona (ES); Buda, Teodora Sandra, 08019 Barcelona (ES); Guerreiro, João, 1600-630 Lisbon (PT); Omaña Iglesias, Jesús Alberto, 08019 Barcelona (ES); Mati , Aleksandar, 17310 Lloret de Mar (ES)
(74) Representative: Herrmann, Uwe

(57) **Abstract**

The present disclosure relates to a system for determining a mental health state of a user and adjusting output content accordingly; the system comprising a monitoring unit, configured to monitor parameters of a user and acquire corresponding data; an analysis unit, configured to extract defined features from data acquired by the monitoring unit; a classification unit, configured to detect a change in a mental health state of the user based on the defined features extracted by the analysis unit and to classify the change in mental state of the user; a control unit configured to adapt or select the content to be output to the user by the output unit based on the detected change of the mental health state of the user classified by the classification unit, and an output unit, configured to output content to a user based on a detected mental health state of the user or a change in the mental health state of the user. Another aspect of the invention relates to a method for determining a mental health state of a person and adjusting output content accordingly.

## Description

The present invention relates to a system and method for determining a mental health state of a user and adjusting content output or presented to the user by the system accordingly. In other words, the interaction of the system with the user is adapted based on a preferably fully automated diagnosis of the system regarding a mental state of the user to ensure exposure of the user to content optimized for the detected mental health state.

As the recent COVID-19 pandemic has doubled the rates of common mental health disorders such as depression and anxiety, there is a huge and growing unmet need to remedy undesired symptoms of mental health conditions in the population. It is estimated that around 1 in 5 (21%) adults experienced some form of depression in early 2021. This is an increase compared to since November 2020 (19%) and more than double that observed before theCOVID-19 pandemic (10%).

This increase of mental health conditions has put a strain on mental health care professionals such as therapists and psychologists whose number have remained constant. In addition to that, contact restricts due to the pandemic have often exacerbated mental health disorders and also posed a hurdle to treatment, as patients could not easily meet mental health care professionals in person.

It is the object of the present invention to alleviate or completely abolish the drawbacks associated with the prior art, In particular, it is an object of the present invention to ensure that all people receive adequate assistance with their mental health conditions without putting an undue strain on mental health care professionals.

A system or method according to the present disclosure can ensure that individual users receive tailored mental health care recommendations and resources (e.g. such as recommended reading, recommended apps focusing ion mindfulness, healthy sleeping etc.) that meet them wherever they are in their clinical trajectory and do so as early as possible in that journey. A highly curated and useralised service of behavioural health and mental wellness enables identification of the appropriate level of care for individuals with the ability to seamlessly move up or down the stepped care continuum of lower and higher intensity interventions to meet their evolving mental health needs.

An aspect of the present disclosure relates to a system for determining a mental health state of a user and adjusting output content accordingly; the system comprising:
- a monitoring unit, configured to monitor parameters of a user and acquire corresponding data;
- an analysis unit, configured to extract defined features from data acquired by the monitoring unit;
- a classification unit, configured to detect a change in a mental health state of the user based on the defined features extracted by the analysis unit and to classify the change in the mental state of the user;
- a control unit configured to adapt or select the content to be output to the user by the output unit based on a detected change of the mental health state of the user classified by the classification unit, and
- an output unit, configured to output content to a user based on a detected mental health state of the user or a change in the mental health state of the user.

Such a system can, for example be employed in the context of e-health, where a user interacts with an electronic device / system, in order to assess his / her mental state and / or strive to improve his / her mental state.

Often a user experiencing, e.g. symptoms of depression turns to a clinician who then assesses the severity of the depression using questionnaires and standard depression scoring methods. Based on this assessment, the clinician suggests a course of action, e.g. the use of a mindfulness app on the user's smart phone to draw aid the user in recognizing positive aspects of life.

Conventionally, the user subsequently regularly consults the clinician to assess whether any progress in the patent's condition is achieved and / or whether the course of action has to be adjusted. The system according to the present disclosure obviates the need for such routine follow-up consultations, because the system automatically provides a diagnosis of the mental state of user, and in particular of changes in the mental state of the user, based on physiological data of the user. In addition to that, the system automatically adjusts the content output to user according to the change in mental state detected. For example, if the system detects a change from mild depression to moderate depression, the system may adapt the content from presenting the user with a mindfulness app to presenting the user with a sleep management and time management app and also prompting the user to spend more time with friends or consult a self-help book.

For example, the output unit configured to output content to a user based on a detected mental health state of the user may be set up to regularly present to the user a program guiding the user through a breathing exercise. Over the course of two weeks of the user performing the breathing exercises, the monitoring unit, comprising one or more sensors, monitors physiological parameters the user, e.g. their stress level, heart rate, pupil dilation, skin conductivity, sleep pattern, the number of steps taken per day etc. The analysis unit then extracts defined features or feature vectors from the data regarding physiological parameters acquired by the monitoring unit detect changes in time of one or more features. For example, the analysis unit may detect that due to breathing exercises the user is more relaxed and thus shows an improved sleep pattern. The classification unit configured to classify the mental state of the user based on the changes detected by the analysis unit thus uses this improvement in relaxation and sleep pattern to classify the mental state of the user in the class "mild anxiety", whereas previously the user showed e.g. moderate anxiety and had difficulties sleeping. The control unit configured to adapt the content output to the user by the output unit based on a detected mental health state of the user classified by the classification unit then adjusts the content presented to the user based on the detected mental health state or the user and / or based on a detected change in the mental health state of the user, e.g. the change from "moderate anxiety" to "mild anxiety". For example, the new content presented to the user may be a mindfulness diary in that the user records positive aspects of daily life rather than a program for breathing exercises.

This way, a system according to the present disclosure can for example automatically assess a user's mental health state based on e.g. physiological data of the user, such as heart rate, pupil dilation, activity profile, sleep time, resting time, response time, skin conductivity etc., and preferably immediately adapt content presented to the user to the detected health state to ensure that the individual user always receives content tailored to the specific needs of the user. This ensures optimal care for the user while reducing the burden on mental health care professionals and reducing the need for face-to-face-interactions.

The monitoring unit can be configured to acquire one or more of: physiological data of the user, data stemming from contextual input from the user, such as ecological momentary assessment data, data from electronic health records, data reflecting a behavior of the user, or any other type of data directly or indirectly indicative of a user's mental health state.

The monitoring unit can comprise one or more sensors that can be different from each other and can be present on one device or multiple separate devices. The sensors can comprise at least one camera, at least one microphone, at least one gyrometer, at least one accelerometer or any other sensor suitable for measuring a physiological state of the user or capturing a parameter indicative of the user's behavior or any other parameter to be monitored by the monitoring unit.

The control unit may be configured to adapt or select the content output to the user by the output unit based on a detected mental health state of the user or a detected change in the mental health state of the user classified by the classification unit. The type of content to be presented can be modified by the control unit. For example, the output unit maybe controlled to initially present a first type of content following a first intervention rationale, e.g. a mindfulness app, tailored to a first detected metal state of the user, and subsequently be controlled to present a second type of content following a second intervention rationale, e.g. a program for breathing exercises, tailored to a second detected metal state of the user.

A system according to the present disclosure may further comprise a prediction unit configured to predict a change in the mental health of the user (and thus a classification result of the classification unit) in the future based on the changes detected by the analysis unit.

In other words, a system according to the present disclosure may leverage change-based features for predicting a change in a user's mental state, in particular in the severity of symptoms of a user's mental condition, and therefore enables a stepped care approach in that content presented to the user matches the detected mental state or change in mental state. For example, the system may predict that the mental state of a user having a mental state classified as "mild anxiety" will in the near future e.g. in two weeks change to "moderate anxiety", because the analysis unit has detected changes in the feature values extracted from the acquired physiological data that indicate reduced physical activity, reduced sleep and rest and reduced social interaction. The predicted change may be classified as a "step up" corresponding to an increase in symptoms and thus in required effectiveness of the content presented, "step down" corresponding to a decrease in symptoms and thus in a milder form of presented content being sufficient or "neutral" if now change has been detected.

The prediction unit and the classification unit can be configured as one single unit, so that the classification unit is not only configured to detect a current change in the mental state of the user but also is configured to predict a future change of the mental state of the user. In other words, the classification unit can identify trends in the mental state of the user and predict future changes of the mental health state.

The analysis unit detecting changes in the features extracted from the data acquired by the monitoring unit and preferably also the classification unit may be realized as a machine-learning based system, such as a neural network, in particular a convolutional neural network.

According to an embodiment, the analysis unit is configured to determine changes in time in defined features extracted from data acquired by the monitoring unit and to determine a change in a mental health state of the user based on the changes of the defined features.

According to an embodiment, the monitoring unit is further configured to receive data reflecting the conscious mental state of the user, such as data stemming from clinical questionnaires and the system further comprises an integration unit, configured to integrate and synchronize e.g. data regarding physiological parameters of the user reflecting the subconscious mental state of the user with the data reflecting the conscious mental state of the user.

Thus, the system may integrate data indicating how the user consciously feels (e.g. questionnaire data) with data indicating the physiological state of the user that the user cannot influence. This increases the accuracy of determining the mental state of the user. The data reflecting the conscious mental state of the user and the physiological data obtained from sensors e.g. present in the user's smart phone or a wearable such as a wrist band or smart watch, may be integrated in a common data base and / or snychronised ,e.g. co-registered with a temporal stamp to link e.g. data obtained at the same time.

In addition to that, the classification unit may comprise a neural network and the data from the integration unit may be used to train the neural network. In this case, for example data from clinical questionnaires, such as a defined depression level, are used as a target and for example physiological data of a patient, data from electronic health records, data stemming from contextual input from the user, such as ecological momentary assessment data, and data reflecting a behavior of the user are input into the classification unit.

The prediction unit may be configured to predict change in the mental health state of the user in the future, e.g. an increase in the severity of anxiety symptoms, based on the changes detected by the classification unit, e.g. a reduction in sleep and activity, and the data reflecting the conscious mental state of the user, e.g. the user reporting an increased feeling of gloom, acquired by the monitoring unit. Thus, the prediction unit may not only leverage the changes in the feature vectors extracted from the physiological data but may also draw on data reflecting the conscious mental state of the user.

The control unit can be configured to adapt the content output to the user by the output unit based on a stepped care paradigm based on a classification result of the classification unit and / or a prediction result of the prediction unit, wherein in the stepped care paradigm defined classes of mental states are associated with defined recommended contents. For example, an increase in symptom severity or a worsening in mental state of the patient may be regarded as a "step up" in the stepped care paradigm and a decrease in symptom severity or an improvement in mental state of the patient may be regarded as a "step down" in the steppped care paradigm

If the control unit is configured to adapt the content output to the user by the output unit based on a stepped care paradigm based on the prediction result of the prediction unit, e.g. an impending worsening of symptoms, the control unit may pre-emptively adjust the content to avoid the worsening of symptoms. In other words, the content may be adjusted based on a predicted change in mental state prior to that change in mental state actually occurring.

The system can be configured to automatically determine the mental health state of the user at a predetermined time-interval, preferably daily, weekly or monthly, wherein the length of the time-interval is determined by the control unit based on a classification result of the classification unit and / or based on a prediction result of the prediction unit. For example, if a worsening of the user's mental state is detected or predicted, the system can determine the mental state more frequently, e.g. daily, to ensure that no changes in the mental state are missed.

In an embodiment of the invention, the output unit, the monitoring unit, the analysis unit, the classification unit, the prediction unit and / or the integration unit are present in one single device, preferably a smart phone. For example, the output unit can be the graphical user interface, the monitoring unit can be the camera of the smart phone that is used to capture a photopletysmogram (PPG) to detect a user's heart rate or to determine pupil dilation of the user. The monitoring unit can also draw on data from a gyroscope present in the smart phone or GPS data to determine the user's activity level. The analysis unit, the classification unit, the prediction unit and / or the control unit may all be realized as a central processing unit of the smart phone. Of course, the monitoring unit can also comprise devices different from a smart phone, such as fitness trackers, smart watches, smart wrist bands and / or other wearables.

The system may further comprise an alert unit configured to output an alert via a device separate from the system (e.g. via a computer terminal present in a physician's office and / or a laptop, tablet or any other electronic device) if the classification result achieved by the classification unit and / or the prediction result by the prediction unit falls within a predefined class, in particular a class associated with a mental condition that has a severity above a defined severity threshold. For example, if "severe depression" has been determined as the mental state of the user, an alert may be displayed on a physician's device, e.g. via a wireless connection or via SMS.

The alert unit may also configured to output an alert if there has been no detectable change in the classification result by the classification unit and / or the prediction result by the prediction unit for a predefined time interval. Thus, for example a physician may be alerted to the fact that an ongoing presentation of content (e.g. the use of a mindfulness app) may not have the desired effect.

Another aspect of the invention relates to a method for determining a mental health state of a user and adjusting output content accordingly; the method preferably employing a system according to the present disclosure, comprising the steps:
- monitoring parameters of the user and acquiring corresponding data by means of a monitoring unit;
- extracting by means of an analysis unit defined features from the data acquired by the monitoring unit;
- detecting by means of a classification unit a change in a mental health state of the user based on the defined features;
- classifying the detected change in the mental state of the user by means of the classification unit into pre-defined classes; and
- adapting or selecting by means of a control unit a content to be presented to the user by the output unit based on the detected change in mental health state of the user classified by the classification unit, and
- outputting content to the user by means of an output unit based on the detected change of the mental health state of the user.

The monitoring step can comprise acquiring one or more of: physiological data of the user, data stemming from contextual input from the user, such as ecological momentary assessment data, data from electronic health records, data reflecting a behavior of the user, or any other type of data directly or indirectly indicative of a user's men-tal health state.

According to an embodiment, the further comprises the step of: by means of the classification unit determining changes in time in the defined features extracted from data acquired by the monitoring unit and determining a change in a mental health state of the user based on the changes of the defined features.

The method may comprise the step of predicting a classification result of the classification unit in the future based on the defined features extracted by the analysis unit or based on changes of the defined features detected by the classification unit.

The method may further comprise the steps of
- acquiring via the monitoring unit data reflecting the conscious mental state of the user, such as data stemming from clinical questionnaires, and physiological parameters of the user using one or more sensors, and
- integrating and synchronizing by means of an integration unit the data regarding physiological parameters of the user reflecting the subconscious mental state of the user with the data reflecting the conscious mental state of the user..

This allows for a more accurate determination of the mental state of the user, because both conscious experience of the user as well as physiological data reflecting the subconscious state of the user are considered.

The prediction step may comprise predicting a change in the mental health state of the user (corresponding to a classification result of the classification unit) in the future based on the changes detected by the analysis unit and the data reflecting the conscious mental state of the user acquired by the monitoring unit. For example, the user can be prompted to provide information on how he/she feels. This way, not only changes detected in the feature vectors extracted by the analysis unit are considered, but also the user's conscious experience is taken account of.

The adapting step performed by the control unit may comprise adapting the content output to the user by the output unit based on a stepped care paradigm based on a classification result of the classification unit, wherein in the stepped care paradigm defined classes of mental states are associated with defined recommended contents. In other words, the control unit may select the content to be output to the user by the output unit based on a stepped care paradigm based on a classification result of the classification unit, wherein in the stepped care paradigm defined classes of mental states are associated with defined recommended contents.

The method may be automatically performed at predetermined time-intervals, preferably daily, weekly or monthly, to determine the mental state of the user, wherein the length of the time-interval is preferably determined by the control unit based on a classification result of the classification unit and / or based on a prediction result of the prediction unit.

The method may further comprise a step of outputting an alert via a separate device if it has been determined that the classification result by the classification unit and / or the prediction result by the prediction unit falls within a predefined class, in particular a class associated with a mental condition that has a severity above a defined severity threshold, or a step of outputting an alert via a separate device if there has been no detectable change in the classification result by the classification unit and / or the prediction result by the prediction unit for a predefined time interval.

As will be apparent to the person skilled in the art, any effects, features or elements described in the context of the system according to the present disclosure equally apply to the method according to the present disclosure even if not explicitly recited herein to reduce redundancy. Similarly, any effects, features or elements recited herein may be taken in isolation or combined in any desired way, as is apparent to the person skilled in the art.

Further effects, features or elements of the present disclosure will become apparent from the following description of embodiments of the invention taking reference to the figures.
Fig. 1 shows a general concept of the operation of a system according to the present disclosure;
Fig. 2 shows a flow chart demonstrating the creation of a system according to the present disclosure and the operation of such a system.
Fig. 3 shows an example of the operation of a system according to the present disclosure, the system being applied to an individual user; and

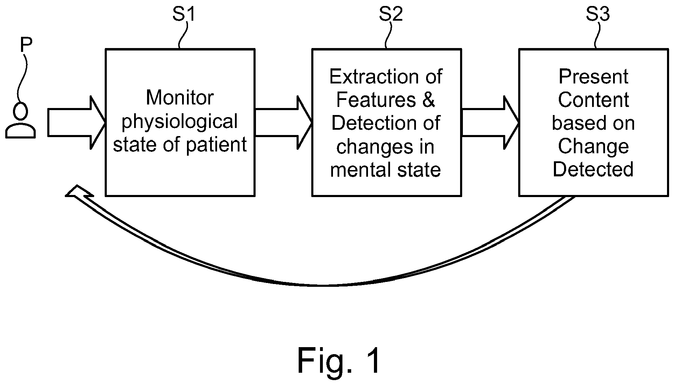

As shown in Fig. 1, in step S1, data reflecting the physiological state of a user P are acquired using a monitoring unit. The physiological parameter measured by the monitoring unit may be blood pressure, pulse rate, skin conductivity, breathing rate, temperature, pupil dilation, gaze direction, or a physiological parameter reflecting the behavior of the user, such as sleep pattern, activity profile, number of steps taken in a day, screen time, rest time, or time taken for social interaction or a movement profile. Any other suitable parameter reflecting the physiological state of the user may be monitored.

The monitoring unit acquires these data preferably passively, without any specific action of the user being required and in some cases without the user even noticing. For example, the monitoring unit may draw on activity data acquired by the user's smart phone or on measurements of the heart rate obtained with a smart watch or the camera of the user's smart phone.

The system and method according to the present disclosure can be used to diagnose many mental health disorders and illnesses, such as, but not limited to: generalized anxiety disorder, panic disorders, phobias, obsessive-compulsive disorders (OCD), post-traumatic stress disorder (PTSD); attention deficient disorder (ADD), and attention deficit hyperactivity disorder (ADHD); depressive disorder, dysthymia, depression in the elderly, postpartum depression; stress or mild depression caused by comorbidity with other health conditions such as strokes, cardiac procedures, cancer treatments, major accidents, and major surgeries and cognitive impairments related to aging. All these disorders and illnesses are described and defined in the Diagnostic and Statistical Manual of Mental Disorders, Fifth Edition (DSM-V).

The data acquired in step 1 is then stored in a database. In step S2, the analysis unit extracts feature vectors from the acquired data and detects changes in the features relative to a previous measurement and / or relative to an expected text book value. Based on this analysis, the mental state of the user is classified, for example drawing on standard categories such as the classes of depression defined in the Diagnostic and Statistical Manual of Mental Disorders, Fifth Edition (DSM-V): "mild", "moderate", "moderately severe"; "severe".

Based on the detected change and classification of the mental state of the user, in step 3, the control unit adapts the content presented to the user as indicated by the feedback loop in Fig. 1. For example, if a worsening in the mental state of the user is detected, a more effective content will be presented to the user, e.g. instead of a mindfulness app, a guided self-help program for depression will be presented.

In other words, Figure 1 illustrates steps performed by a system or in a method according to the present invention, as follows:
S1: The first step is monitoring which involves capturing behaviors and / or physiological data and / or cognitive signals leveraging novel technologies, such as sensors from smart devices, in order to
In S2 detect changes with regards to standard clinical measurements such as from Diagnostic and Statistical Manual of Mental Disorders (DSM-5), which in practice is used by clinicians and researchers to diagnose and classify mental disorders, based on change-based features thus enabling
In S3 the model of stepped care, where higher or lower intensity content is delivered based on detected changes.

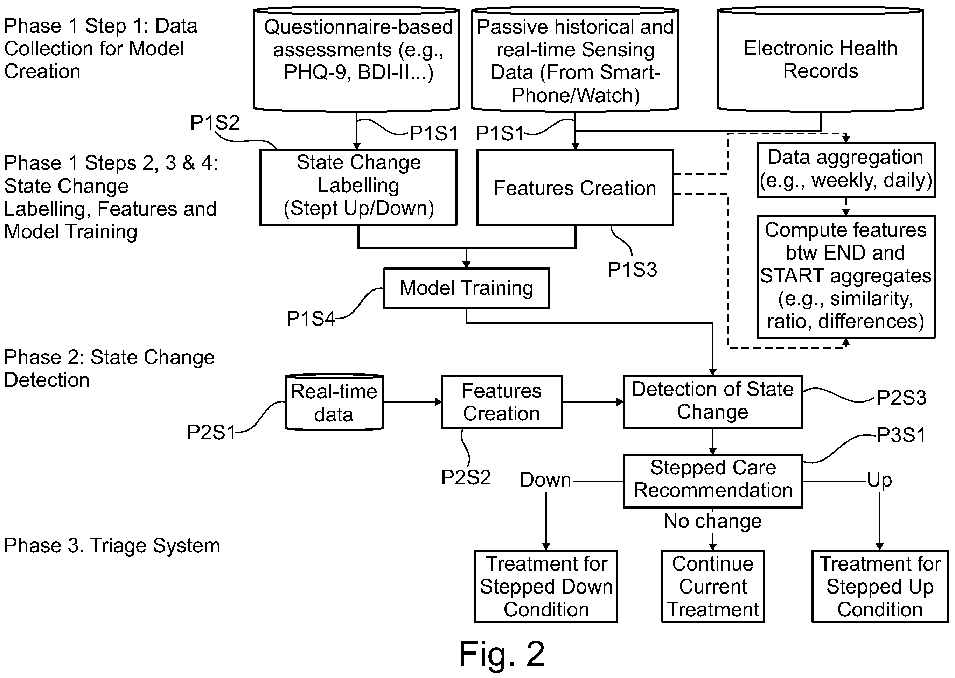

Fig. 2 shows a flow chart demonstrating the creation of a system according to the present disclosure and the operation of such a system.

Generally, in Fig. 2, phase 1 shows the steps required for training a convolutional neural network (CNN) to be capable of detecting state changes in the feature vectors extracted from physiological real-time data acquired from an individual user.

Phases 2 and 3 show the steps after the CNN has been trained, where the system and method according to the present disclosure are used to automatically diagnose the mental state of the individual user based on physiological real-time data acquired from an individual user and adapt the content presented to the user in a stepped care paradigm (also referred to as a triage system in Fig. 2).

Phase 1 in that the CNN (also referred to as the model) is trained, in this example comprises four steps:
In step 1 of phase 1 (Step P1S1 in Fig. 2) data are collected for the creation of the model: In this step, the data from relevant sources, such as from e.g. smartphone sensors, wearables, electronic health records, questionnaire data, text notes - users thoughts diaries or clinicians notes -, voice recordings etc. are collected and stored into a database.

The data collection may include but is not limited to a part of individual's state assessment with questionnaires (e.g. the PHQ-9 BDI-II questionnaires for depression as outlined in the DSM-V) and a part of passive data from sensors (e.g. step count, sleep duration, heart rate, screen time, stationary time, etc.). The results of the questionnaires are collected at least twice, separated by a period of time over which the system will need to assess the state change. During a desired data collection period, the passive data is collected with a certain time granularity (e.g. daily).

In step P1S1, historical data about users are coupled with the ground-truth information about the symptom severity. Historical data about users can contain passive data collected from users' devices such as smartphone sensors or smartwatches. This data can be used as a proxy or to represent day to day behaviours (such as location, physical activity, sleep, light, social interactions) and physiological states (such as through ECG, heart rate, heart rate variability, electrodermal activity, screen swiping patterns, reaction times, etc). Ground truth information about symptom severity that is necessary for training a CNN can be collected through clinical assessments, or from Electronic Health Records (EHR). In addition, it can be collected by questionnaire-based self-reports such as Ecological Momentary Assessments (EMA), or standard scales such as PHQ-9 (depression), wellbeing (WHO-5),anxiety (GAD-7), etc. that are known from e.g. the DSM-V.

Typically data will be aggregated in different categories for different time intervals (hourly, daily, weekly) from these data sources. For instance, data obtained from smartphone sensors can be aggregated into the following features or any other desired features based on availability:
- Daily step count (pedometer)
- Daily activity summaries: percentage time stationary, walking, automotive, running. Total minutes stationary, walking, automotive, running.
- Daily screen time
- Hourly activity counts
- Hourly screen counts
- Daily time sleeping
- Swiping patterns

In step 2 of phase 1 (SP1S2 in Fig. 2) state change labelling in performed: In this step, the questionnaire data reflecting the conscious mental state of an individual user is classified depending on the results into a severity level (e.g. for PHQ-9 the result is assigned to a severity level according to the score: 0-4="none", 5-9="mild", 10-14="moderate", 15-19="moderately severe", 20-27="severe").

Then, the results of the same individual from two different points in time are compared and the time period between the two assessments is labelled as follows: "step up" if the second result is in a higher severity level than the first one; "no change" if both results are within the same severity level; and "step down" if the second result is in a lower severity level than the first one.

In other words, to determine the change in mental state, in step 2 of phase 1 the data from questionnaires is used. In order to compute a change, the individuals need to report their mental state (e.g.. symptoms severity) via the same assessment method, e.g. the same questionnaire, at least two times with a certain time separation between them (e.g. two weeks).

For instance, for the questionnaire PHQ-8, the severity of depression symptoms is defined based on the value of the result with the following ranges : 0-4="none", 5-9="mild", 10-14="moderate", 15-24="severe". The state change is labeled as "step up" if the individual increased the severity range within a certain time period (i.e. between one and the subsequent measurement). Conversely, "step down" reflects the situation in which the symptoms became less severe in a certain time period i.e. the symptom severity range is lower after a certain time period (i.e. from one to the subsequent measurement), or "no change" if an individual remained in the same symptom severity range.

In step 3 of phase 1 (SP1S3 in Fig. 2) features and / or feature vectors are created from the collected data: In this step, the collected data is transformed into features and / or feature vectors that capture the differences between the physiological data reflecting e.g the individual's behaviour between two different moments in time. In that sense, the data is aggregated at a certain time interval level (e.g. daily level or weekly level) over the period of time that the system is meant to detect the state change. For that purpose, a set of functions that combine the aggregated data at the end of the time period and the aggregated data at the beginning of the time period is applied, resulting in the features for the detection model. These functions include but are not limited to similarity measures, ratios and differences between the aggregated data at the start and the end of the period.

In this example, the feature creation process is divided into two stages. In the first stage the passive data is aggregated in order to capture the physiological data reflecting behaviour or cognitive states at the beginning and at the end of the evaluated period. In that sense, a certain time period around the starting point and the end point is defined (e.g. the week before the first questionnaire measurement and the week before the second questionnaire). Then, the collected data from passive sensors are aggregated over those points in time by computing a set of variables, such as:
- Average across days
- Total sum
- Standard deviation across days
- Variance across days
- Minimum value
- Maximum value
- Highest N (e.g. 3) values
- Lowest N (e.g. 3) values
- Average swiping speed
- Total distance covered in swiping
- Inefficiency

In the second stage, the set of variables are transformed into the change-based features that are used in the following step for modeling. These features are designed to reflect behaviour and cognitive state changes between the start of the period and the end of the period. The functions that are used in order to compute these features include, but are not limited to the following:
Similarity measure features may be obtained: For example, for each of the highest N and lowest N types of variables (e.g. the number of steps taken), a similarity measure feature is computed. This similarity measure is, for instance the cosine similarity between the N highest/lowest values at the start and the N highest/lowest values at the end of the period. The N values in both cases are ordered by the value. For instance, if N=3 (i.e. the lowest three or the highest three values are selected from a given period, in this example one week), the values in the first week are: Mon=3 (corresponding to e.g. three steps being taken that day), Tue=4, Wed=2, Thu=7, Fri=1, Sat=6, Sun=5 and the values in the second week are: Mon=2, Tue=6, Wed=3, Thu=4, Fri=0.5, Sat=1.5, Sun=7. Then, the similarity measure on the lowest N=3 is computed between the vector for the first week: (1,2,3) corresponding to the three lowest values in the first week and the vector for the second week: (0.5,1.5,2) corresponding to the three lowest values in the second week.

Additionally or alternatively, regression slope features may be obtained: The daily values of each data type can be used used to compute the regression slopes. For each data type, a linear regression is fitted using all the daily values available, using the day position within the period from the start to the end as the independent variable and the value as the dependent variable. The slope of the fitted regression is saved as a feature.

Additionally or alternatively, ratio features may be obtained: For each of the aggregated variables (e.g. averages, total sum, standard deviation, total distance covered swiping, etc.), the ratio between the value at the end of the period and the value at the beginning of the period can be computed and saved as a feature.

Additionally or alternatively, difference features may be obtained: For each of the aggregated variables (e.g. averages, total sum, standard deviation, total distance covered swiping, etc.), the difference between the value at the end of the period and the value at the beginning of the period is computed and saved as a feature..

In step 4 of phase 1 (P1S4 in Fig. 2) the CNN / model is trained using the data obtained in steps P1S1-P1S3: In this step, the features created are used to train a state change model. There are two variants of how step P1S4 may be performed, in the first one a model for step up and a separate model for step down are built and in the second one a single multiclass model to detect step up, down or no change is built.

In other words, a machine learning model (CNN) is trained using the change-based features above and the stepped score ("step ip", "step down" or "no change") as target.

In phase 2, the model trained in Phase 1 Step 4 is applied to real-time data obtained from an individual user in order to detect a potential change in severity of symptoms for the user.

In step 1 of phase 2 (P2S1 in Fig. 2) data is collected reflecting the mental state of an individual user, e.g. physiological data obtained from passive sensors. This step is thus similar to the data collection step from phase 1. The data from multiple individuals is collected and stored in the database. However, in this case the data from the questionnaires is collected only once at the start of the interaction of the user with the system to establish a mental state as a starting point and for the rest of the period during that the system or method is used by the user, only data acquired by sensors that monitor the users physiological state without requiring the user to actively make statements are used.

In step 2 of phase 2 (P2S2 in Fig. 2), the same features computed at phase 1 are created on the new data collected during this phase. The set of features created and the length of time period preferably match those used in phase 1.

In step 3 of phase 2 (P2S3 in Fig. 2), the model trained at phase 1 is used to detect change in the mental state of the user based on the acquired data. The features created at the previous step serve as input to the model and the state change over the time period is classified into step-up, step-down or no change.

In step 1 of phase 3 (P3S1 in Fig. 2), the system decides based on the detected state change whether the individual needs to be presented with a milder or more intensive content or the same content should be continued to be displayed.

Based on the detection, a triage system is employed for delivering tailored interventions: If a step up in severity is detected, the user can be referrred to a different content based on the updated severity in symptoms. In case of digital mental healthcare if a step up change is detected, the user can be referred to a specific digital content e.g. Cognitive Behavioural Therapy digitally provided via an App.

If a step down in severity is detected, the user can be referred to a different, milder content. In case of digital mental healthcare, the user can be referred to a preventative or a wellness app (e.g. an app providing mindfulness, sleep programmes, assistance in building healthy habits, cognitive restructuring for self-esteem, low mood, worry etc.).

If no change is detected, in case of no or mild symptoms, the user would continue to be presented with the same content, whereas if the ongoing content did not result in an expected improvement, a clinician could be informed.

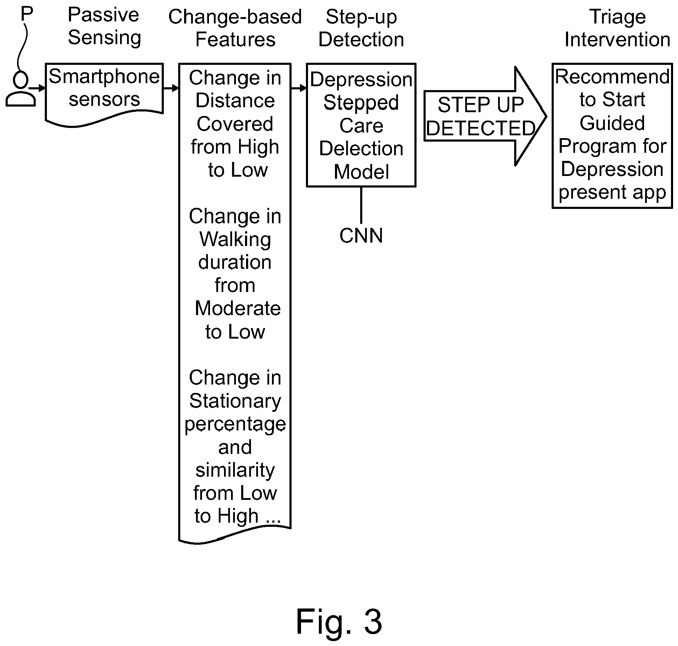

In following with reference to Fig. 3 an example is given in that the invention is applied to an individual user.

In the example, user P is using an application for the smartphone for managing stress at a sub-clinical level, which collects data reflecting the users physiological state and behaviour from the smartphone sensors, such as a gyrometer, a camera etc. The data is acquired passively and / or continuously without the user having to take any specific action.

In this example, the model detects a step-up in the severity of symptoms for user P based on the change-based features computed between Week 0 and Week 2 and recommends the user to start a guided program for depression. The content displayed to the user is thus an app delivering a guided program for depression. The data collection and features creation for this example are described in more detail below. Initially, data are collected and aggregated, examples of which are given below. Based on the acquired smartphone sensors data, the system aggregates the data into the following smartphone sensors and event logs:
- 'data_accumulatedSteps': the number of steps during a segment
- 'data_averageActivePace': Average pace at which the user moved,
- 'data_currentCadence': The rate at which steps are taken, measured in steps per second ,
- 'data_currentPace': The last pace of the user, measured in seconds per meter,
- 'data_distance': Total distance covered. Provided by CoreMotion on iOS and calculated internally on Android in order to provide an estimation.
- 'data_duration': Number of seconds the segment covered.
- 'data_floorsAscended': the number of floors ascended by the user during a segment
- 'data_floorsDescended': the number of floors descended by the user during a segment.
- 'data_activityCountFor24h': the total number of events detected
- , 'data_longestInterruptedRest': a feature allowing for a brief gap to no count as an interruption of the rest.
- 'data_longestStationary": the longest period of lack of phone physical movemen or the longest longest period screen is turned off.

Table 1 and 2 illustrates a numerical examples of data collected for user P during week 0 and week 2, respectively. The tables illustrate the raw data collected using the sensor library. The features shown below are merely illustrative and not limiting.

**Table 1. Data collected for user P during Week 0**

| Day | A | B | C | D | E | F | G | H | I | J | K |
|---|---|---|---|---|---|---|---|---|---|---|---|
| 1 | 1148.0 | 1.0 | 0.0 | 0.0 | 829.0 | 86399. 0 | 2.0 | 2.0 | 612.0 | 25200. 0 | 26806. 0 |
| 2 | 11778.0 | 0.0 | 0.0 | 0.0 | 8619.0 | 86399.0 | 6.0 | 7.0 | 776.0 | 21600.0 | 24784.0 |
| 3 | 11473. 0 | 0.0 | 0.0 | 0.0 | 7921.0 | 86399. 0 | 4.0 | 8.0 | 1042.0 | 18000. 0 | 19221. 0 |
| 4 | 18748. 0 | 0.0 | 0.0 | 0.0 | 13904. 0 | 86399. 0 | 6.0 | 7.0 | 942.0 | 18000. 0 | 28672. 0 |
| 5 | 19805. 0 | 0.0 | 0.0 | 0.0 | 14164. 0 | 86399. 0 | 6.0 | 13.0 | 842.0 | 21600. 0 | 24516. 0 |
| 6 | 20141. 0 | 0.0 | 0.0 | 0.0 | 14804. 0 | 86399. 0 | 8.0 | 4.0 | 1003.0 | 21600. 0 | 28198. 0 |
| 7 | 20750. 0 | 0.0 | 0.0 | 0.0 | 14850. 0 | 86399. 0 | 6.0 | 17.0 | 1011.0 | 21600. 0 | 28300. 0 |

**Table 2. Data collected for User P during Week 2**

| Day | A | B | C | D | E | F | G | H | I | J | K |
|---|---|---|---|---|---|---|---|---|---|---|---|
| 15 | 5644.0 | 0.0 | 0.0 | 0.0 | 4134.0 | 86399. 0 | 8.0 | 12.0 | | 28800. 0 | 989.0 |
| 16 | 14301. 0 | 0.0 | 0.0 | 0.0 | 10695. 0 | 86399. 0 | 4.0 | 5.0 | 897.0 | 79200. 0 | 22882. 0 |
| 17 | 17999. 0 | 0.0 | 0.0 | 0.0 | 13141. 0 | 86399. 0 | 4.0 | 3.0 | 1051.0 | 25200. 0 | 25351 . 0 |
| 18 | 7928.0 | 0.0 | 0.0 | 0.0 | 5876.0 | 86399. 0 | 2.0 | 3.0 | 1061.0 | 28800. 0 | 13346. 0 |
| 19 | 20023. 0 | 0.0 | 0.0 | 0.0 | 13910. 0 | 86399. 0 | 7.0 | 7.0 | 929.0 | 10800. 0 | 19053. 0 |
| 20 | 24265.0 | 0.0 | 0.0 | 0.0 | 17810.0 | 86399.0 | 7.0 | 6.0 | 790.0 | 14400.0 | 32658.0 |
| 21 | 11489. 0 | 0.0 | 0.0 | 0.0 | 8548.0 | 86399. 0 | 1.0 | 1.0 | 885.0 | 21600. 0 | 13296. 0 |

Wherein the columns in tables 1 and 2 correspond to the following features:
- A = 'data_accumulatedStep'
- B = 'data_averageActivePace'
- C = 'data_currentCadence'
- D = 'data_currentPage'
- E = 'data_distance'
- F = 'data_duration'
- G = 'data_floorsAscended'
- H = 'data_floorsDescended'
- I = 'data_activityCountFor24h'
- J = 'data_longestlnterruptedRest'
- K = 'data_longestStationary'

Ground-truth information about the change in the conscious mental health state of the user has been captured by the PHQ-8 questionnaire, reported by individuals / users three times: baseline, at the 2-week point and at the 4-week point.

The state change is defined as "*step up*" if the individual has a level of severity in the second questionnaire result that is higher than the first one, "*step down*" if the level of severity in the second questionnaire is lower than the first one or "*no-change*" if both reports are the same severity range. For PHQ-8, the severity of depression symptoms is classified based on a score arising from the questionnaire that is classified according to the following classes: 0-4="none", 5-9="mild", 10-14="moderate", 15-24="severe".

In this example, user P answers a PHQ-8 questionnaire at onboarding and scores 4, classified as 'None'. The user answers the same questionnaire week 2 and week 4, scoring 8 and 10 respectively, corresponding to initially mild then moderate depression. In this particular case, the CNN model identified indications of the mental state of the user decreasing corresponding to a "step up" in the content that should be presented to the user, since the user stepped up in symptoms severity.

In this particular example, the following aggregation methods for the features were used in Phase 1 Step 1:
- Absolute change in mean between weeks 0 and 2 (or 2 and 4)
- Absolute change in mean between week 0, week1 and week2 (or 2, 3 and 4)
- Relative change in mean/standard deviation between weeks 0 and 2 (or 2 and 4)
- Relative change in mean/standard deviation between weeks 0, week1 and week2 (or 2, 3 and 4)
- Normalized regression slope over 2 weeks
- Cosine similarity between weeks 0 and 2 (or 2 and 4) for subsample of:
   ∘ top 3 daily values
   ∘ bottom 3 daily values

This generated ∼100 features (# sensors x # aggregation methods). Following, the correlations between features vs targets were explored and 14 features with statistically significant correlation to the targets ("step up ", "step down","no change") were indentified to be used for predictive modeling. The features used in this example are:
- 'data_distance_similarity_low_vals': cosine similarity between weeks 0 and 2 of the bottom 3 daily values of distance,
- 'data_daytimeActivity_similarity_low_vals': cosine similarity between weeks 0 and 2 of the bottom 3 daily values of daytime activity,
- 'distance_covered_wk_mean_change': relative change in mean between weeks 0 and 2 of the total distance daily,
- 'data_distance_mean_diff': absolute change in mean between weeks 0 and 2 of the total distance daily,,
- 'data_floorsAscended_mean_diff: absolute change in mean between weeks 0 and 2 of the floors ascended daily,
- 'data_stepSize_wk_mean_change': relative change in mean between weeks 0 and 2 of the step size,
- 'inefficiency_similarity_low_vals': cosine similarity between weeks 0 and 2 of the bottom 3 daily values of inefficiency of swiping movements,
- 'data_longestStationary_similarity': cosine similarity between weeks 0 and 2 of daily values of longest time stationary between 5pm and next 5pm,
- 'data_longestStationary_similarity_low_vals': cosine similarity between weeks 0 and 2 of the bottom 3 daily values of longest time stationary between 5pm and next 5pm,
- 'mean_walking_duration_diff_wk': absolute change in mean between weeks 0 and 2 of the total walking duration daily,
- 'mean_stationary_perc_diff_wk': absolute change in mean between weeks 0 and 2 of the percentage walking duration daily,
- 'ratio_stationary_perc_diff_wk': ratio between the mean in weeks 0 and 2 of the percentage time stationary,
- 'mean_longesstationary_stationary_diff_wk': absolute change in mean between weeks 0 and 2 of the longest time stationary between 5pm and next 5pm,
- 'ratio_longesstationary_stationary_diff_wk': ratio between the mean in weeks 0 and 2 of the longest time stationary between 5pm and next 5pm

Table 3 illustrates some change based features computed for this user P. In this example, cosine similarity was used to determine similarity between feature vectors. A value of 1 indicates that there was no change.

**Table 3. Change-based features computed between Week 4 and Week 2**

| A | B | C | D | E | F | G | H | I | J | K | L | M | N |
|---|---|---|---|---|---|---|---|---|---|---|---|---|---|
| 0.94 | 0.99 | 0.91 | 139. 71 | 0.57 | 0.98 | 0.99 | 0.99 | 0.90 | 431. 57 | - 0.01 | 0.99 | 624 9.02 | 1.13 |

Wherein the columns in tables 1 and 2 correspond to changes in the following features:
- A= 'data_distance_similarity_low_vals',
- B = 'data_daytimeActivity_similarity_low_vals',
- C = 'distance_covered_wk_mean_change',
- D = 'data_distance_mean_diff',
- E = 'data_floorsAscended_mean_diff',
- F = 'data_stepSize_wk_mean_change',
- G = 'inefficiency_similarity_low_vals'
- H = 'data_longestStationary_similarity',
- I = 'data_longestStationary_similarity_low_vals',
- J = 'mean_walking_duration_diff_wk',
- K = 'mean_stationary_perc_diff_wk',
- L = 'ratio_stationary_perc_diff_wk',
- M = 'mean_longesstationary_stationary_diff_wk',
- N = 'ratio_longesstationary_stationary_diff_wk'

As can be seen in table 3 and illustrated in Fig. 3, changes were detected in fields D, J an M corresponding to changes in the distance covered by the user, the walking duration and the stationary time of the user. In summary, the user changed to a more sedentary life style over the course of the four weeks that have been investigated.

Data regarding these 14 change features and the questionnaire data (as ground truth data) were then used to train a convolutional neural network to predict changes in the mental state of the user based on detected feature changes.

As an example of a machine learning model or CNN, XGBoost can be used. In this example, the 14 features listed above were used with data collected from all users as an input for the XGBoost model and the step-up depression score as a target.

XGBoost represents an optimized implementation of gradient boosted decision trees, yet any other machine learning algorithm or CNN can be employed for this task.

After training, the CNN detects based on the detected feature changes shown in Table 3 that the user is highly likely to be experiencing a step up in their symptoms and the triage system recommends the user to start a guided program for depression. Suitable content, e.g. an app guiding the user through a self-help programm can be displayed on the user's smart phone.

The identified increase in symptom severity detected by the CNN was confirmed by the questionnaire results of the user.

## Claims

1. System for determining a mental health state of a user and adjusting output content accordingly; the system comprising:
- a monitoring unit, configured to monitor parameters of a user and acquire corresponding data;
- an analysis unit, configured to extract defined features from data acquired by the monitoring unit;
- a classification unit, configured to detect a change in a mental health state of the user based on the defined features extracted by the analysis unit and to classify the change in mental state of the user;
- a control unit configured to adapt or select the content to be output to the user by the output unit based on the detected change of the mental health state of the user classified by the classification unit, and
- an output unit, configured to output content to a user based on a detected mental health state of the user or a change in the mental health state of the user.

2. System according to claim 1, further comprising a prediction unit, configured to predict a change in the mental health state of the user in the future based on the features extracted by the analysis unit or based on changes in the features extracted by the analysis unit.

3. System according to claim 1 or 2, wherein the monitoring unit is configured to acquire one or more of: physiological data of the user, data stemming from contextual input from the user, such as ecological momentary assessment data, data from electronic health records, data reflecting a behavior of the user, or any other type of data directly or indirectly indicative of a user's mental health state.

4. System according to one of the preceding claims, wherein the classification unit is configured to determine changes in time in defined features extracted from data acquired by the monitoring unit and to determine a change in a mental health state of the user based on the changes of the defined features.

5. System according to one of the preceding claims, wherein the monitoring unit is configured to receive data reflecting the conscious mental state of the user, such as data stemming from clinical questionnaires, and to monitor physiological parameters of the user using one or more sensors, wherein the system further comprises an integration unit, configured to integrate and synchronize the data regarding physiological parameters of the user reflecting the subconscious mental state of the user with the data reflecting the conscious mental state of the user.

6. System according to one of the preceding claims, wherein the prediction unit is configured to predict a change in the mental health state of the user in the future based on changes in the features detected by the analysis unit and data reflecting a conscious mental state of the user acquired by the monitoring unit.

7. System according to one of the preceding claims, wherein the control unit is configured to adapt the content output to the user by the output unit based on a stepped care paradigm based on a classification result of the classification unit, wherein in the stepped care paradigm defined classes of mental states and / or defined classes of change of mental states are associated with defined recommended contents.

8. System according to one of the preceding claims, wherein the system is configured to automatically determine the mental health state of the user and / or a change therein at a predetermined time-interval, preferably daily, weekly or monthly, wherein the length of the time-interval is determined by the control unit based on a classification result of the classification unit and / or based on a prediction result of the prediction unit.

9. System according to one of the preceding claims, wherein the output unit, the monitoring unit, the analysis unit, the classification unit and / or the prediction unit are present in one single device, preferably a smart phone.

10. System according to one of the preceding claims, further comprising an alert unit configured to output an alert via a device separate from the system if the classification result by the classification unit and / or the prediction result by the prediction unit falls within a predefined class, in particular a class associated with a mental condition that has a severity above a defined severity threshold.

11. System according to claim 10, wherein the alert unit is configured to output an alert if there has been no detectable change in the classification result by the classification unit and / or the prediction result by the prediction unit for a predefined time interval.

12. Method for determining a mental health state of a user and adjusting output content accordingly; the method preferably employing a system of one of claims 1-11, comprising the steps:
- monitoring parameters of the user and acquiring corresponding data by means of a monitoring unit;
- extracting by means of an analysis unit defined features from the data acquired by the monitoring unit;
- detecting by means of a classification unit a change in a mental health state of the user based on the defined features;
- classifying the detected change in the mental state of the user by means of the classification unit into pre-defined classes; and
- adapting or selecting by means of a control unit a content to be presented to the user by the output unit based on the detected change in mental health state of the user classified by the classification unit, and
- outputting content to the user by means of an output unit based on the detected change of the mental health state of the user.

13. Method according to claim 12, wherein the monitoring step comprises acquiring one or more of: physiological data of the user, data stemming from contextual input from the user, such as ecological momentary assessment data, data from electronic health records, data reflecting a behavior of the user, or any other type of data directly or indirectly indicative of a user's mental health state.

14. Method according to claim 12 or 13, further comprising the step of:
by means of the classification unit determining changes in time in the defined features extracted from data acquired by the monitoring unit and determining a change in a mental health state of the user based on the changes of the defined features.

15. Method according to one of claims 12-14, further comprising the step of predicting a change in the mental health state of the user in the future based on the defined features extracted by the analysis unit or based on changes of the defined features detected by the analysis unit.

16. Method according to one of claims 12-15, further comprising the steps of
- acquiring via the monitoring unit data reflecting the conscious mental state of the user, such as data stemming from clinical questionnaires, and physiological parameters of the user using one or more sensors, and
- integrating and synchronizing by means of an integration unit the data regarding physiological parameters of the user reflecting the subconscious mental state of the user with the data reflecting the conscious mental state of the user.

17. Method according to one of the claims 12-16, wherein the prediction step comprises predicting a change in the mental health state of the user in the future based on the changes detected by the analysis unit and the data reflecting the conscious mental state of the user acquired by the monitoring unit.

18. Method according to one of the claims 12-17, wherein the adapting step performed by the control unit comprises adapting the content output or to be output to the user by the output unit based on a stepped care paradigm based on a classification result of the classification unit, wherein in the stepped care paradigm defined classes of mental states and / or classes of change of mental state are associated with defined recommended contents.

19. Method according to one of the claims 12-18, wherein the method is automatically performed at predetermined time-intervals, preferably daily, weekly or monthly, to determine the mental state of the user or a change therein, wherein the length of the time-interval is determined by the control unit based on a classification result of the classification unit and / or based on a prediction result of the prediction unit.

20. Method according to one of the claims 12-19, further comprising a step of outputting an alert via a separate device if it has been determined that the classification result by the classification unit and / or the prediction result by the prediction unit falls within a predefined class, in particular a class associated with a mental condition that has a severity above a defined severity threshold, or a step of outputting an alert via a separate device if there has been no detectable change in the classification result by the classification unit and / or the prediction result by the prediction unit for a predefined time interval.
